# EUROPEAN PATENT APPLICATION

(11) **EP 2 995 933 A1**
(43) Date of publication of application: **16.03.2016**
(21) Application number: 14184817.6
(22) Date of filing: 15.09.2014
(51) Int. Cl.: G01N 21/65, B82Y 5/00, B82Y 15/00, B82Y 35/00, C12Q 1/68, G01J 3/44, G01N 33/487, G02B 6/122

(54) **Improved nanopore plasmonic analyser**

(71) Applicant: Base4 Innovation Ltd, Cambridge, Cambridgeshire CB3 0FA (GB)
(72) Inventor: The designation of the inventor has not yet been filed
(74) Representative: Lau, Sarah Jane

(57) **Abstract**

An apparatus for investigating a molecule comprises:
• a substrate;
• first and second chambers arranged either side of the substrate;
• at least one nanopore in said substrate linking the first and second chambers;
• at least one pair of nanostructures, located on the substrate, capable of generating an electromagnetic field when caused to undergo localised surface plasmon resonance;
• a source of incident electromagnetic radiation for inducing localised surface plasmon resonance in the nanostructures:
• a controller for causing the molecule to pass from the first to the second chambers via the inlet(s) and outlet(s) and through the electromagnetic field and
• a detector for detecting Raman-scattered radiation produced by the molecules as they pass through the electromagnetic field.

It is characterised in that the nanostructures are juxtaposed opposite each other around the nanopore and have morphology in a plane parallel to that of the substrate comprising sectors of a circle having a from 30 to 150 degree central angle. The apparatus is especially suitable for investigating biomolecules; in particular for sequencing the amino acids of a protein or the nucleotides of a nucleic acid such as naturally occurring or synthetic RNA or DNA.

## Description

This invention relates to an improved nanopore plasmonic apparatus for investigating molecules using Raman spectroscopy. It is especially useful for investigating biomolecules for example the sequencing of nucleic acids such as DNA and RNA.

In our previously published patent application WO 2009/030953 we have disclosed an apparatus for investigating a molecule which comprises a nano-perforated substrate provided with plasmonic structures juxtaposed within or adjacent the outlet of the nanopores. In this apparatus, the plasmonic structures define detection windows comprising an electromagnetic field within which the analyte molecule (optionally labelled) is in induced to fluoresce or Raman-scatter photons in characteristic way by interaction with incident light. The photons so generated are then detected remotely, multiplexed and converted into a data-stream whose information content is characteristic of the molecular structure of the analyte itself. This information can then be recovered from the data-stream using computational algorithms embodied in corresponding hardware or software programmed into a microprocessor integral therewith or in a separate computing apparatus attached thereto. In one useful embodiment the analyte is a nucleic acid and the apparatus is used to determine the sequence of its constituent nucleotide bases by electrophoretically translocating the nucleic acid base-by-base through the nanopores of the substrate into the detection window(s).

US 2005/084912 discloses another apparatus for examining inter alia the sequence of nucleotide bases in a DNA sample as it translocates through a nanopore. Here, the apparatus suitably comprises a microscope detector consisting of an optical system comprising a nanolens assembly provided with one or more plasmon resonant particles. The optical system is arranged to be moveable and separate from the substrate bearing the nanopore.

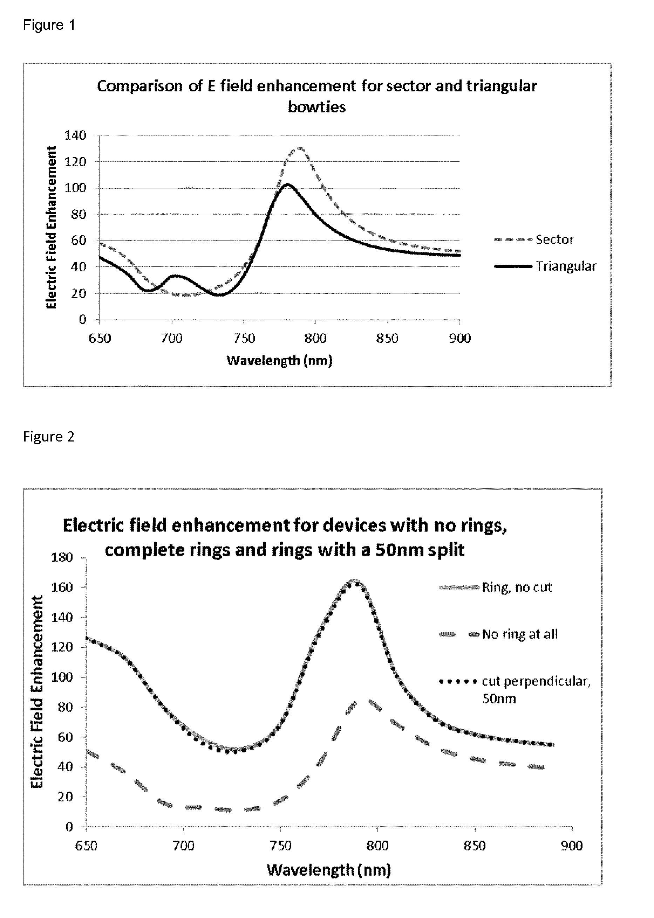

US 2008/0239307 teaches a surface-enhanced Raman-scattering (SERS) method and a corresponding apparatus for sequencing polymeric biomolecules such as DNA, RNA or proteins. In the method disclosed, metallic nanostructures are caused to undergo plasmonic resonance thereby creating an electrical field enhancement near their surface which in turn can improve the Raman-scattering cross-section of any biomolecule located nearby. In Figure 1 of this application an arrangement is shown in which the nanostructures are arranged on the surface of a nanoperforated substrate so that the biomolecule may be caused to translocate both through the nanopore and between the nanostructures.

WO 2011/076951 discloses a similar system for manipulating and analysing biomolecules using nanostructures and associated forces created by plasmon resonance. In one embodiment, it is taught that the system can also comprise a SERS detector.

Finally, JP 2010/0243267 and WO 2012/043028 disclose apparatuses in which biopolymers are caused to translocate through a perforated substrate coated with a thin metal film. Raman-scattering of light from the biopolymer is then analysed using a Raman detector.

We have now found that the sensitivity and therefore the accuracy of our apparatus can be improved by specifying the morphology of the plasmonic structures used in association with the nanopore(s) in the substrate. This is especially important when then apparatus relies on detecting Raman-scattering produced by SERS interaction between the molecule being investigated (the analyte), the plasmon resonator and the electromagnetic field.

Whilst we are aware that various shapes of plasmonic structures have generally been discussed and theoretically treated in the prior art (see for example Optical Society of America (2014) OCIS code 180.4243 and Phys. Rev. B85 045434 (2012)), we are unaware of pairs of the particular nanostructures described below being used for any detection duty including biomolecule sequencing apparatuses.

Thus according to the present invention there is provided an apparatus for investigating a molecule comprising:
- a substrate;
- first and second chambers arranged either side of the substrate;
- at least one nanopore in said substrate linking the first and second chambers;
- at least one pair of nanostructures located on the substrate, capable of generating an electromagnetic field when caused to undergo localised surface plasmon resonance;
- a source of incident electromagnetic radiation for inducing localised surface plasmon resonance in the nanostructures:
- a controller for causing the molecule to pass from the first to the second chambers via the inlet(s) and outlet(s) and through the electromagnetic field and
- a detector for detecting Raman-scattered radiation produced by the molecules as they pass through the electromagnetic field
characterised in that the nanostructures are juxtaposed opposite each other around the nanopore and have a morphology in a plane parallel to that of the substrate comprising sectors of a circle having a from 30 to 150 degree central angle.

In an embodiment of the invention the apparatus further comprises a microprocessor or computer for analysing a signal produced by the detector.

The apparatus of the present invention can be used for a number of different duties. For example, in one embodiment it can be used to detect biological pathogens or poisons in drinking water, cooling fluids or air; to detect residual pesticides and pollutants in aqueous extracts taken from soils and foodstuffs and to detect narcotics, explosives or their residua on hard surfaces, clothing or the like. In these duties the apparatus may be positioned in a guard location with the triggering of an alarm being the intended output.

In another embodiment the apparatus can be used to investigate the nature and disposition of the constituent monomer units of an organic polymer. A preferred duty in this respect is the investigation of the internal structure of biomolecules such as proteins and nucleic acids; especially the determination of the nature of the amino acids in proteins or nucleotides in nucleic acids.

A most preferred duty of all is the sequencing of the nucleotides in natural-occurring or synthetic DNA or RNA. In this duty the apparatus is for example used to sequence polynucleotide fragments greater than 50 nucleotides, preferably greater than 150 nucleotides, most preferably greater than 250 nucleotides long.

The substrate employed in the apparatus of the invention is suitably made of a dielectric material such as glass, silicon or silicon nitride and in one embodiment is a sheet of such material. However the use of a composite sheet for example in which the dielectric material provides the surface(s) bearing the nanostructure(s) is also envisaged. Typically the substrate is 5-200nm thick preferably 20 to 200nm. In one embodiment the substrate is a membrane made of silicon, silicon oxide, silicon nitride or materials with equivalent properties. In another the membrane can be mounted on a support made of dielectric support. In yet another, the substrate may include a layer which can function as a mirror for the incident electromagnetic radiation.

The substrate is perforated with one or more nanopores which may disposed in any arrangement including in a regular array across the whole or a part thereof. Typically, each nanopore has a diameter in the range 0.5 to 100 nm, preferably from 0.5 to 50nm most preferably from 0.5 to 10nm. Useful results can be obtained using nanopores whose diameters lie in the range 0.5 to approximately 5nm. In addition to being nanoporous, one or more surfaces of the substrate can be further provided with wells, cavities or channels of nanodimensions.

Turning to the pair(s) of nanostructures, these are juxtaposed opposite each other around the outlet of the nanopore(s) and are sized and arranged so that localised surface plasmons, as opposed to surface plasmon polaritons, can be induced therein. An advantage of this arrangement is that, by judicious choice of size, the nanostructures can be tuned to produce optimum plasmonic field intensity and density for a given wavelength of the incident electromagnetic radiation. In practice, this means that the each nanostructure should have a maximum dimension of less than 1 micron, preferably in the range 30 to 500 nanometres, most preferably in the range 50 to 250 nanometres. In one embodiment, the pairs of nanostructures are disposed in a regular disposition on a surface of the substrate and about the outlets of the nanopores. In another embodiment, each nanostructure is spaced apart from its pair by less than 10 nanometres in order to induce a strong electromagnetic field which causes enhancement of any Raman-scattering signal obtained from the molecule being investigated. Hereinafter the space which this electromagnetic field occupies is referred to as the 'detection window'.

The nanostructures themselves are typically fabricated from metals or dielectric materials coated with metal. Metals which can be employed are those capable of undergoing plasmon resonance to a significant extent, for example, gold, silver, copper, aluminium, platinum, palladium, molybdenum and chromium and alloys thereof. Preferably, the metal used will be gold, silver, copper or an alloy thereof. To enhance the performance of the apparatus further, the nanostructure may have attached to its surface binding sites which are specifically adapted to capture the particular target molecule and enhance the characteristic spectroscopic emissions being sought. Such reactive groups can work by any chemical or physical means; for example when detecting say a signal characteristic of a bacterial pathogen, e.g. the Legionella bacterium, the reactive group can comprise a polynucleotide probe adapted to bind to certain unique base-pair sequences in the bacterium DNA by hybridisation.

The nanostructures employed in the apparatus of the present invention are characterised by having a morphology which in a plane parallel to that of the substrate corresponds to the sector of a circle. Suitably the central angle associated with the sector is in the range 30 to 150 degrees or 30 to degrees; for example 30 to 60 or 60 to 90 degrees. Such nanostructures are herein after referred to as 'sector nanostructures'. By the term sector of a circle is meant both a precise geometric sector of a circle or slight deviations therefrom which still show superiority over the prior art 'bow-tie' arrangements of nanostructure pairs wherein the morphology in the plane parallel to that of the substrate corresponds to that of a triangular wedge. The precise morphology of the sector nanostructures can for example be determined by scanning electron microscopy. In one embodiment of the invention each pair of sector nanostructures are enclosed within a separate annulus of plasmonic material of one of the types described above. In another this annulus may be comprised of a pair of half annuli; e.g. annuli separated along an axis perpendicular to that defined by the foci of the sectors. This half annuli arrangement has the additional advantage that they are easier to fabricate; in particular they do not suffer fracturing problems when the 'lift-off' processing method described in the next paragraph is employed.

The sector nanostructures are suitably prepared by first coating the substrate with a metallic film, then masking up the coated product and finally etching away the remaining exposed metal and substrate with a chemical or an ion beam to leave discrete the nanostructure pairs arranged about the nanopores. Methods for carrying out such preparations are well known in the art. In one embodiment the nanostructures may be prepared using lift-off processing. This involves patterning a resist such as poly methyl methacrylate on the substrate by exposure to electron beam irradiation, followed by developing and then metallisation. The resist film is then dissolved, removing the metal everywhere except where the film was developed.

In a typical working form, the apparatus comprises one or more nanoperforated substrates and their associated nanostructures arranged in a vessel so as to divide the latter into first and second chambers for respectively providing and receiving molecules of the analyte. The apparatus is also provided with a controller for causing molecules of the analyte to translocate from the first chamber to the second chamber via the nanopore(s) and the detection window(s). Typically this controller works by establishing a gradient between the chambers along which the analyte molecule is then induced to pass. Such gradients may include for example those generated by variations in osmotic pressure, or magnetic field or by using chemical reactions to generate a thermodynamic or entropic gradient. However, in one preferred embodiment the gradient is created by an electric field produced by applying a potential difference between the contents of the first and second chambers. The analyte molecule is then caused to translocate by electrophoresis, or by dielectrophoresis, in the case of uncharged, polarisable molecules. When the analyte molecule comprises a protein or a nucleic acid the controller employs electrophoresis. In one embodiment, the direction of the potential difference can be reversed; for example to remove molecules of the analyte from the substrate after it has been used so that the apparatus can be cleaned. In another embodiment, the means for establishing this potential difference and the detector may be connected via a feedback loop so that, once the detector detects an expected Raman signal, the direction of the electric field can be varied with time to hold the analyte molecule in the detection window, fine tune its velocity therethrough or allow resequencing.

The potential difference referred to above can be achieved by locating a first electrode in the first chamber, locating a second electrode, of opposite polarity, in the second chamber and then applying a voltage therebetween.

The remainder of the apparatus comprises a high-intensity source of incident electromagnetic radiation, for example a laser or the like, a detector for detecting the characteristic Raman-scattering and any necessary ancillary optics and electrical circuitry. Either or both of the source of incident electromagnetic radiation and the detector can be continuously associated with some or all of the nanostructures and the detection windows. Alternatively, the nanostructures and the detection windows can be scanned using for example a movable microscope or raster arrangement. The Raman-scattered radiation detected by the detector can in principle be any scattering which is uniquely characteristic of one or more vibrational modes of the analyte and can be derived from single- or multi-photon events.

The nature of the detector employed in the apparatus is not critical and can suitably comprise for example a photodetector, a single photon avalanche diode, an electron-multiplying charge-coupled apparatus or a complementary metal oxide semiconductor apparatus. The detector can additionally be attached to a microprocessor, PC and/or an alarm to process the signal derived therefrom.

Whilst it is envisaged that the whole apparatus can be of unitary design it can also advantageously consist of two components for example a permanent housing in which the source of incident electromagnetic radiation, the detector and optics are located and a disposable cartridge comprising a vessel containing the chambers, substrate and nanostructures linked together by microfluidic pathways. In this case the vessel then can be made of for example glass, silicon or plastic. If the apparatus is of unitary design and is used as a chemical sensor e.g. for guard duties, it may be provided with a wireless transmitter to send the signal from the detector back to a central location. The apparatus can then be positioned remotely in which case it may employ a stand-alone power source such as battery if so desired.

The invention is now illustrated by the following.

### Example 1

Electric field enhancements were calculated for two nanostructure of the same size one comprising a pair of sixty degree sectors of a circle and the other comprising sixty degree equilateral triangles. The results shown in Figure 1 demonstrate the degree of electromagnetic field enhancement produced by the former arrangement over the latter. This translates into a corresponding enhancement of the Raman-scattering of nucleotides of a DNA molecule translocating through a nanopore (4^{th} power of the field).

### Example 2

Figure 2 also shows that employing a split-ring annulus as described above does not materially affect that part of the electromagnetic field enhancement due to it.

## Claims

1. An apparatus for investigating a molecule comprising:
• a substrate;
• first and second chambers arranged either side of the substrate;
• at least one nanopore in said substrate linking the first and second chambers;
• at least one pair of nanostructures, located on the substrate,, capable of generating an electromagnetic field when caused to undergo localised surface plasmon resonance;
• a source of incident electromagnetic radiation for inducing localised surface plasmon resonance in the nanostructures:
• a controller for causing the molecule to pass from the first to the second chambers via the inlet(s) and outlet(s) and through the electromagnetic field and
• a detector for detecting Raman-scattered radiation produced by the molecules as they pass through the electromagnetic field
**characterised in that** the nanostructures are juxtaposed opposite each other around the nanopore and have a morphology in a plane parallel to that of the substrate comprising sectors of a circle having a from 30 to 150 degree central angle.

2. An apparatus as claimed in claim 1 **characterised in that** each pair of nanostructures are surrounded by a separate annulus of material capable of also undergoing plasmon resonance

3. An apparatus as claimed in claim 2 **characterised in that** the annulus is comprised of a pair of half annuli separated along an along an axis perpendicular to that defined by the foci of the sectors.

4. An apparatus as claimed in claim 1 **characterised in that** the substrate further comprises a mirror layer.

5. An apparatus as claimed in claim 1 **characterised in that** the central angle is from 45 to 90 degrees.

6. An apparatus as claimed in claim 1 **characterised in that** each nanostructure is spaced apart from its pair by less than 10nm.

7. An apparatus as claimed in claim 1 **characterised in that** each nanostructure has a maximum diameter from 50 to 250nm.

8. An apparatus as claimed in claim 1 **characterised in that** the controller comprises a pair of electrodes respectively located in the first and second chambers.

9. An apparatus as claimed in claim 8 **characterised in that** the controller and detector form part of a feedback loop.

10. An apparatus as claimed in claim 1 **characterised in that** the detector is a detector for detecting the nucleotides of a nucleic acid or the amino acids of a protein.

11. An apparatus as claimed in claim 10 **characterised by** being suitable for sequencing a nucleic acid or an amino acid.

12. An apparatus as claimed in claim 1 **characterised by** further comprising PC, microprocessor or alarm.

13. An apparatus a claimed in claim 1 **characterised in that** the first and second chambers and the substrate are provided in the form of a disposable microfluidic chip.

14. An apparatus as claimed in claim 13 **characterised by** further comprising electrodes in the first and second chambers.

15. A disposable microfluidic chip **characterised in that** it is adapted for use in an apparatus according to claim 12.
